# EUROPEAN PATENT APPLICATION

(11) **EP 1 876 448 A1**
(43) Date of publication of application: **09.01.2008**
(21) Application number: 05021530.0
(22) Date of filing: 30.09.2005
(51) Int. Cl.: G01N 33/68, C07K 14/705, C07K 14/47, C07K 16/28, C07K 16/18

(54) **Method and analytical reagents for identifying therapeutics using biomarkers responsive to thiazolidinediones.**

(71) Applicant: DIGILAB BioVisioN GmbH, 30625 Hannover (DE)
(72) Inventor: Budde, Petra, 30655 Hannover (DE); Rose, Horst, 31303 Burgdorf (DE); Zucht, Hans-Dieter, 30539 Hannover (DE); Appel, Annette, 31515 Wunsdorf (DE)
(74) Representative: Schnappauf, Georg

(57) **Abstract**

The present invention relates to methods for screening for therapeutics on the basis of biomarkers responsive to treatment with thiazolidinediones, in particular therapeutics having activities analogous to thiazolidinediones. Such therapeutics may be useful, for example, in therapy of Type II diabetes, insulin resistance, obesity, impaired glucose tolerance, metabolic syndrome (syndrome x), cardiovascular diseases, atherosclerosis and dyslipidemia. Furthermore, the invention relates to methods for testing whether an individual will benefit from treatment with the afore mentioned therapeutics.
The biomarkers are selected from Fn14, HMGA1, apelin, fragments and variants thereof.

SEQUENCE LISTING

<110>  BioVision AG

<120>  METHOD FOR IDENTIFYING THERAPEUTICS USING BIOMARKERS RESPONSIVE TO
THIAZOLIDINEDIONES

<130>  412-35

<160>  14

<170>  PatentIn version 3.1

<210>  1
<211>  129
<212>  PRT
<213>  Homo sapiens

<400>  1

Met Ala Arg Gly Ser Leu Arg Arg Leu Leu Arg Leu Leu Val Leu Gly
1               5                   10                  15

Leu Trp Leu Ala Leu Leu Arg Ser Val Ala Gly Glu Gln Ala Pro Gly
            20                  25                  30

Thr Ala Pro Cys Ser Arg Gly Ser Ser Trp Ser Ala Asp Leu Asp Lys
        35                  40                  45

Cys Met Asp Cys Ala Ser Cys Arg Ala Arg Pro His Ser Asp Phe Cys
    50                  55                  60

Leu Gly Cys Ala Ala Ala Pro Pro Ala Pro Phe Arg Leu Leu Trp Pro
65                  70                  75                  80

Ile Leu Gly Gly Ala Leu Ser Leu Thr Phe Val Leu Gly Leu Leu Ser
                85                  90                  95

Gly Phe Leu Val Trp Arg Arg Cys Arg Arg Arg Glu Lys Phe Thr Thr
            100                 105                 110

Pro Ile Glu Glu Thr Gly Gly Glu Gly Cys Pro Ala Val Ala Leu Ile
        115                 120                 125

Gln

<210>  2
<211>  129
<212>  PRT
<213>  Mus musculus

<400>  2

Met Ala Pro Gly Trp Pro Arg Ser Leu Pro Gln Ile Leu Val Leu Gly
1               5                   10                  15

Phe Gly Leu Val Leu Met Arg Ala Ala Ala Gly Glu Gln Ala Pro Gly

20                  25                  30

Thr Ser Pro Cys Ser Ser Gly Ser Ser Trp Ser Ala Asp Leu Asp Lys
        35                  40                  45

Cys Met Asp Cys Ala Ser Cys Pro Ala Arg Pro His Ser Asp Phe Cys
    50                  55                  60

Leu Gly Cys Ala Ala Ala Pro Pro Ala His Phe Arg Leu Leu Trp Pro
65                  70                  75                  80

Ile Leu Gly Gly Ala Leu Ser Leu Val Leu Val Leu Ala Leu Val Ser
                85                  90                  95

Ser Phe Leu Val Trp Arg Arg Cys Arg Arg Arg Glu Lys Phe Thr Thr
            100                 105                 110

Pro Ile Glu Glu Thr Gly Gly Glu Gly Cys Pro Gly Val Ala Leu Ile
        115                 120                 125

Gln

<210>  3
<211>  106
<212>  PRT
<213>  Homo sapiens

<400>  3

Ser Glu Ser Ser Ser Lys Ser Ser Gln Pro Leu Ala Ser Lys Gln Glu
1               5                   10                  15

Lys Asp Gly Thr Glu Lys Arg Gly Arg Gly Arg Pro Arg Lys Gln Pro
            20                  25                  30

Pro Val Ser Pro Gly Thr Ala Leu Val Gly Ser Gln Lys Glu Pro Ser
        35                  40                  45

Glu Val Pro Thr Pro Lys Arg Pro Arg Gly Arg Pro Lys Gly Ser Lys
    50                  55                  60

Asn Lys Gly Ala Ala Lys Thr Arg Lys Thr Thr Thr Thr Pro Gly Arg
65                  70                  75                  80

Lys Pro Arg Gly Arg Pro Lys Lys Leu Glu Lys Glu Glu Glu Glu Gly
                85                  90                  95

Ile Ser Gln Glu Ser Ser Glu Glu Glu Gln
            100                 105

<210>  4
<211>  106

<212>  PRT
<213>  Mus musculus

<400>  4

Ser Glu Ser Gly Ser Lys Ser Ser Gln Pro Leu Ala Ser Lys Gln Glu
1               5                   10                  15

Lys Asp Gly Thr Glu Lys Arg Gly Arg Gly Arg Pro Arg Lys Gln Pro
            20                  25                  30

Pro Val Ser Pro Gly Thr Ala Leu Val Gly Ser Gln Lys Glu Pro Ser
        35                  40                  45

Glu Val Pro Thr Pro Lys Arg Pro Arg Gly Arg Pro Lys Gly Ser Lys
    50                  55                  60

Asn Lys Gly Ala Ala Lys Thr Arg Lys Val Thr Thr Ala Pro Gly Arg
65                  70                  75                  80

Lys Pro Arg Gly Arg Pro Lys Lys Leu Glu Lys Glu Glu Glu Glu Gly
                85                  90                  95

Ile Ser Gln Glu Ser Ser Glu Glu Glu Gln
            100                 105


<210>  5
<211>  77
<212>  PRT
<213>  Homo sapiens

<400>  5

Met Asn Leu Arg Leu Cys Val Gln Ala Leu Leu Leu Leu Trp Leu Ser
1               5                   10                  15

Leu Thr Ala Val Cys Gly Gly Ser Leu Met Pro Leu Pro Asp Gly Asn
            20                  25                  30

Gly Leu Glu Asp Gly Asn Val Arg His Leu Val Gln Pro Arg Gly Ser
        35                  40                  45

Arg Asn Gly Pro Gly Pro Trp Gln Gly Gly Arg Arg Lys Phe Arg Arg
    50                  55                  60

Gln Arg Pro Arg Leu Ser His Lys Gly Pro Met Pro Phe
65                  70                  75


<210>  6
<211>  77
<212>  PRT
<213>  Mus musculus

<400>  6

Met Asn Leu Arg Leu Cys Val Gln Ala Leu Leu Leu Leu Trp Leu Ser

1                   5                   10                  15

Leu Thr Ala Val Cys Gly Val Pro Leu Met Leu Pro Pro Asp Gly Thr
            20                  25                  30

Gly Leu Glu Glu Gly Ser Met Arg Tyr Leu Val Lys Pro Arg Thr Ser
        35                  40                  45

Arg Thr Gly Pro Gly Ala Trp Gln Gly Gly Arg Arg Lys Phe Arg Arg
    50                  55                  60

Gln Arg Pro Arg Leu Ser His Lys Gly Pro Met Pro Phe
65                  70                  75

<210>   7
<211>   57
<212>   PRT
<213>   Mus musculus

<400>   7

Glu Gln Ala Pro Gly Thr Ser Pro Cys Ser Ser Gly Ser Ser Trp Ser
1               5                   10                  15

Ala Asp Leu Asp Lys Cys Met Asp Cys Ala Ser Cys Pro Ala Arg Pro
            20                  25                  30

His Ser Asp Phe Cys Leu Gly Cys Ala Ala Ala Pro Pro Ala His Phe
        35                  40                  45

Arg Leu Leu Trp Pro Ile Leu Gly Gly
    50                  55

<210>   8
<211>   56
<212>   PRT
<213>   Mus musculus

<400>   8

Glu Gln Ala Pro Gly Thr Ser Pro Cys Ser Ser Gly Ser Ser Trp Ser
1               5                   10                  15

Ala Asp Leu Asp Lys Cys Met Asp Cys Ala Ser Cys Pro Ala Arg Pro
            20                  25                  30

His Ser Asp Phe Cys Leu Gly Cys Ala Ala Ala Pro Pro Ala His Phe
        35                  40                  45

Arg Leu Leu Trp Pro Ile Leu Gly
    50                  55

<210>   9
<211>   60
<212>   PRT

<213>  Mus musculus

<400>  9

Glu Gln Ala Pro Gly Thr Ser Pro Cys Ser Ser Gly Ser Ser Trp Ser
1               5                   10                  15

Ala Asp Leu Asp Lys Cys Met Asp Cys Ala Ser Cys Pro Ala Arg Pro
            20                  25                  30

His Ser Asp Phe Cys Leu Gly Cys Ala Ala Ala Pro Pro Ala His Phe
        35                  40                  45

Arg Leu Leu Trp Pro Ile Leu Gly Gly Ala Leu Ser
    50                  55                  60

<210>  10
<211>  61
<212>  PRT
<213>  Mus musculus

<400>  10

Glu Gln Ala Pro Gly Thr Ser Pro Cys Ser Ser Gly Ser Ser Trp Ser
1               5                   10                  15

Ala Asp Leu Asp Lys Cys Met Asp Cys Ala Ser Cys Pro Ala Arg Pro
            20                  25                  30

His Ser Asp Phe Cys Leu Gly Cys Ala Ala Ala Pro Pro Ala His Phe
        35                  40                  45

Arg Leu Leu Trp Pro Ile Leu Gly Gly Ala Leu Ser Leu
    50                  55                  60

<210>  11
<211>  23
<212>  PRT
<213>  Mus musculus

<400>  11

Gly Arg Pro Lys Lys Leu Glu Lys Glu Glu Glu Glu Gly Ile Ser Gln
1               5                   10                  15

Glu Ser Ser Glu Glu Glu Gln
            20

<210>  12
<211>  26
<212>  PRT
<213>  Mus musculus

<400>  12

Val Pro Leu Met Leu Pro Pro Asp Gly Thr Gly Leu Glu Glu Gly Ser
1               5                   10                  15

Met Arg Tyr Leu Val Lys Pro Arg Thr Ser
            20                  25

<210>  13
<211>  15
<212>  PRT
<213>  Mus musculus

<400>  13

Leu Pro Pro Asp Gly Thr Gly Leu Glu Glu Gly Ser Met Arg Tyr
1               5                   10                  15

<210>  14
<211>  13
<212>  PRT
<213>  Mus musculus

<400>  14

Gln Arg Pro Arg Leu Ser His Lys Gly Pro Met Pro Phe
1               5                   10

## Description

### Field of the invention

The present invention is in the field of pharmaceutical research tools. Specifically, the present invention provides methods for screening for therapeutics on the basis of biomarkers responsive to treatment with thiazolidinediones, in particular therapeutics having activities analogous to thiazolidinediones. Such therapeutics may be useful, for example, in therapy of Type II diabetes, obesity, impaired glucose tolerance, metabolic syndrome (syndrome x), cardiovascular diseases, polycystic ovary syndrome, atherosclerosis and dyslipidemia. Through their immunomodulatory and anti-inflammatory actions, thiazolidinediones and other PPAR-gamma agonists may prove to be effective in treating diseases unrelated to insulin resistance, such as autoimmune (e.g., multiple sclerosis), atopic (e.g., asthma, atopic dermatitis) and other inflammatory diseases (e.g., psoriasis, ulcerative colitis). Furthermore, the invention relates to methods for testing whether an individual will benefit from treatment with the afore mentioned therapeutics.

### Background of the invention

Type II diabetes, impaired glucose tolerance, obesity, metabolic syndrome, cardiovascular diseases and atherosclerosis are all diseases interconnected to each other.
The metabolic syndrome is a cluster of the most dangerous cardiovascular disease (CVD) risk factors: diabetes or pre-diabetes, abdominal obesity or obesity, changes in cholesterol and hypertension. Abdominal obesity or obesity is mainly responsible for the rising prevalence of metabolic syndrome. Obesity is associated with increased circulating levels of several acute-phase proteins and inflammatory cytokines that contribute to a state of low-grade inflammation causally linked to insulin resistance. Insulin resistance is present in the majority of patients with metabolic syndrome and is also strongly associated with a risk of cardiovascular diseases and Type II diabetes.

Adipose tissue or fat cells secrete or release a diverse range of protein factors that are collectively named "adipokines". These adipokines are involved in lipid metabolism, insulin sensitivity, the alternative complement system, vascular hemostasis, blood pressure regulation and angiogenesis, as well as the regulation of energy balance. In addition, there is a growing list of adipokines involved in inflammation (TNF-alpha, IL-1, IL-6, IL-8, IL-10, transforming growth factor, nerve growth factor) and the acute-phase response (plasminogen activator inhibitor-1 (PAI-1), haptoglobin, serum amyloid A). An increased level of PAI-1 contributes to a prothrombotic state, while low levels of adiponectin correlate with worsening of metabolic risk factors.

Insulin resistance is also one of the principal effects underlying Type II diabetes. Peroxisome-proliferator-activated receptor gamma (PPAR-gamma) agonists, including glitazones and thiazolidinediones (TZDs), are powerful insulin sensitizers.

PPAR including the subtypes PPAR-alpha, PPAR-beta (also termed PPAR delta), and PPAR-gamma are members of the nuclear receptor superfamily that bind specific DNA response elements and in response to ligand binding, result in the activation of several genes. PPAR heterodimerize with the retinoid X receptor (RXR) and bind to specific DNA response elements (PPRE). Upon ligand binding to PPAR, the receptor experiences a conformational change that results in activation of gene transcription. It was suggested that PPAR are critical regulators in metabolic pathways involving energy storage, and thus are potential targets for therapeutics against disorders such as obesity (Kliewer, et al., Recent Progress in Hormone Research, 2001, 56: 239-263). PPAR-gamma is known to play an important role in the regulation of glucose and lipid homeostasis as well as in adipocyte differentiation (Willson, et al., Journal of Medicinal Chemistry, 2000, 43: 527-550).

The physiologic role and tissue expression of PPAR-alpha, -beta and -gamma are described in the following table:

| PPAR | Physiologic role | Tissue expression |
|---|---|---|
| PPAR-alpha | Lipid catabolism | Brown fat, Liver, Kidney, Heart, |
| | Wound healing | Diaphragm, Skeletal muscle, |
| | Perxoisome Proliferator | Immune system, Intestine, Retina |
| PPAR-beta (=PPAR-delta) | Cell proliferation | Ubiquitous |
| | Adipocyte differentiation | Skeletal and cardiac muscle |
| | Myelination | |
| | Embryonic implantation | |
| PPAR-gamma | Adypocyte differentiation | Adipose, Colon, Cecum, Immune |
| | Lipid storage | system, Vasculature, Bladder, |
| | Macrophage maturation | Spleen |
| | Inflammation | |

The potential therapeutic indications of PPAR-agonists are as follows:

| Agonists for the following PPARs | Potential indications |
|---|---|
| PPAR-alpha | Dyslipedemia (decreasing triglycerides/increasing HDL) |
| PPAR-beta | Obesity (decreasing gamma-mediated fluid accumulation and weight gain) |
| PPAR-gamma | Type II diabetes (improving insulin sensitivity) |
| PPAR-dual (alpha/gamma) | Diabetes and Dyslipidemia |
| PPAR-pan (alpha/beta/gamma) | Diabetes, Dyslipidemia, Obesity [metabolic syndrome] |

Thiazolidinediones such as Rosiglitazone, Troglitazone, Pioglitazone, and MCC-555 and non-thiazolidinedione PPAR-gamma agonist, such as RWJ-348260 cause a major redistribution of body fat with a decrease in visceral and hepatic fat content resulting in an increase in insulin sensitivity. The glucose lowering effects of thiazolidinediones are similar to those achieved with the well known drug metformin, useful for treating insulin resistance, Type II diabetes and related disorders. Thiazolidinediones improve the pro-coagulant state, show benefits in improving endothelial dysfunction, reduce inflammatory cytokines and increase adiponectin levels. A new picture of thiazolidinediones is emerging, that suggests that the greatest benefit for the thiazolidinediones might be to directly influence plasma lipids and systemic inflammatory markers, ameliorating atherogenesis/ atherosclerosis and preventing coronary artery restenosis in diabetic subjects. However, the response of patients to particular thiazolidinediones are quite variable, and 20-30% of patients are classified as non-responders.

It was an object of the present invention to provide an effective and sensitive screening method for the identification of substances having therapeutic potential in the treatment of the above-mentioned diseases. Specifically, it was an object to provide methods which allow the screening for substances having functional activities similar to those of thiazolidinediones and biomarkers useful in such screening methods.

A further object of the present invention was to provide methods which allow predicting whether an individual is responsive to treatment with a substance having functional activities similar to those of thiazolidinediones.

This object is achieved by the subject matter of the claims.

In one aspect the invention relates to a method for identifying a therapeutic comprising the step of contacting a cell with a candidate substance and determining the level of at least one peptide and/or protein selected from the group consisting of Fn14, HMGA1, Apelin, fragments thereof, and variants thereof.

Preferably, the cell used in the method of the invention is responsive to treatment with a thiazolidinedione and/or expresses a PPAR-gamma receptor. In a particularly preferred embodiment the cell is an adipocyte, adipose tissue or cell type having a similar secretory profile as adipocytes, in particular monocytes and macrophages.

The candidate substance may be a compound or a composition of compounds.

Preferably, the therapeutic is a substance having at least one functional property of a thiazolidinedione or having analogous activity to a thiazolidinedione.

In a preferred embodiment, an increase in the level of said at least one peptide and/or protein and/or a decrease in the level of said at least one peptide and/or protein if a cell has been contacted with said candidate substance relative to if a cell has not been contacted with said candidate substance indicates that the candidate substance is a therapeutic useful in the treatment of a disease, in particular a disease associated with a PPAR-gamma receptor. In a further preferred embodiment, an increase in the level of said at least one peptide and/or protein if a cell has been contacted with said candidate substance relative to if a cell has not been contacted with said candidate substance indicates that the candidate substance has at least one functional property of a thiazolidinedione and/or is useful in the treatment of a disease treatable with a thiazolidinedione and/or associated with a PPAR-gamma receptor.

In particular embodiments of the method of the invention, said contacting takes place in vitro in a sample or within a living organism.

In further embodiments of the method of the invention, the level of said at least one peptide and/or protein is compared with the level of said at least one peptide and/or protein in a reference, wherein in said reference preferably a cell (i) has not been contacted with said candidate substance, and/or (ii) has been contacted with a thiazolidinedione or a combination of a thiazolidinedione and said candidate substance, and/or (iii) has been contacted with said candidate substance, a thiazolidinedione or a combination of a thiazolidinedione and said candidate substance for a different period of time and/or in a different concentration.

In one embodiment of the method of the invention, said contacting takes place within a living organism by administering said candidate substance to said living organism and the level of said at least one peptide and/or protein is compared with the level of said at least one peptide and/or protein in at least one further living organism (i) to which said candidate substance has not been administered, and/or (ii) to which a thiazolidinedione or a combination of a thiazolidinedione and said candidate substance has been administered, and/or (iii) in which said contacting with said candidate substance, a thiazolidinedione or a combination of a thiazolidinedione and said candidate substance took place for a different period of time, and/or (iv) to which a different dose of said candidate substance has been administered, and/or (v) to which said candidate substance has been administered by different means of administration.

In a further aspect the invention relates to a method for determining whether a living organism is responsive to treatment with a substance having at least one functional property of a thiazolidinedione comprising the step of administering said substance to said living organism and determining the level of at least one peptide and/or protein selected from the group consisting of Fn14, HMGA1, Apelin, fragments thereof, and variants thereof in a sample obtained from said living organism, wherein said sample is preferably selected from the group consisting of blood, serum, plasma, urine, adipose tissue, CSF, bone marrow, brain tissue, white blood cells, vascular endothelial cells, microglia cells.

In the method according to this aspect of the invention, an increase in the level of said at least one peptide and/or protein and/or a decrease in the level of said at least one peptide and/or protein in a living organism to which said substance has been administered relative to a living organism to which said substance has not been administered indicates that said substance is effective in treating a disease, in particular a disease associated with a PPAR-gamma receptor. Preferably, an increase in the level of said at least one peptide and/or protein in a living organism to which said substance has been administered relative to a living organism to which said substance has not been administered indicates that said substance is effective in the treatment of a disease treatable with a thiazolidinedione and/or associated with a PPAR-gamma receptor.

In particular embodiments, said level of said at least one peptide and/or protein is compared with the level of said at least one peptide and/or protein in at least one further living organism (i) to which said substance has not been administered, and/or (ii) to which a thiazolidinedione or a combination of a thiazolidinedione and said substance has been administered, and/or (iii) which has been subjected to said substance, a thiazolidinedione or a combination of a thiazolidinedione and said substance for a different period of time, and/or (iv) to which a different dose of said substance has been administered, and/or (v) to which said substance has been administered by different means of administration.

In preferred embodiments according to all aspects of the invention, said determining comprises determining the level of Fn14 and/or at least one fragment thereof and/or at least one variant thereof and/or HMGA1 and/or at least one fragment thereof and/or at least one variant thereof and/or Apelin and/or at least one fragment thereof and/or at least one variant thereof.

According to the invention, the term "Fn14, fragments thereof, and variants thereof" relates to any protein or peptide comprising the sequence of a Fn14 protein, a fragment thereof or a variant thereof as defined herein. In preferred embodiments of the present invention, the Fn14 protein, fragment thereof or variant thereof corresponds to or is derived from the Fn14 protein naturally, i.e. without any recombinant intervention, encoded by the cell used in the methods of the invention. Alternatively, a cell used in the methods of the invention may encode a Fn14 protein, fragment thereof or variant thereof which is heterologous to said cell. Preferably, the Fn14 protein, fragment thereof or variant thereof corresponds to or is derived from human or murine Fn14 and preferably corresponds to or is derived from a Fn14 protein comprising an amino acid sequence according to SEQ ID NO:1 or 2 of the sequence listing. A fragment of Fn14 preferably corresponds to amino acid residues 28 to 84, 28 to 83, 28 to 87 or 28 to 88 of a Fn14 protein, preferably of murine Fn14, more preferably of the sequence shown in SEQ ID NO:2 of the sequence listing, or corresponds to amino acid residues of a Fn14 protein corresponding to the afore-mentioned amino acid residues in a homology comparison. In a particularly preferred embodiment, a fragment of Fn14 has an amino acid sequence selected from the group consisting of SEQ ID NO:7 to 10 of the sequence listing.

According to the invention, the term "HMGA1, fragments thereof, and variants thereof" relates to any protein or peptide comprising the sequence of a HMGA1 protein, a fragment thereof or a variant thereof as defined herein. In preferred embodiments of the present invention, the HMGA1 protein, fragment thereof or variant thereof corresponds to or is derived from the HMGA1 protein naturally, i.e. without any recombinant intervention, encoded by the cell used in the methods of the invention. Alternatively, a cell used in the methods of the invention may encode a HMGA1 protein, fragment thereof or variant thereof which is heterologous to said cell. Preferably, the HMGA1 protein, fragment thereof or variant thereof corresponds to or is derived from human or murine HMGA1 and preferably corresponds to or is derived from a HMGA1 protein comprising an amino acid sequence according to SEQ ID NO:3 or 4 of the sequence listing. A fragment of HMGA1 preferably corresponds to amino acid residues 73 to 95 of a HMGA1 protein, preferably of murine HMGA1, more preferably of the sequence shown in SEQ ID NO:4 of the sequence listing, or corresponds to amino acid residues of a HMGA1 protein corresponding to the afore-mentioned amino acid residues in a homology comparison. In a particularly preferred embodiment, a fragment of HMGA1 has the amino acid sequence according to SEQ ID NO:11 of the sequence listing.

According to the invention, the term "Apelin, fragments thereof, and variants thereof" relates to any protein or peptide comprising the sequence of an Apelin protein, a fragment thereof or a variant thereof as defined herein. In preferred embodiments of the present invention, the Apelin protein, fragment thereof or variant thereof corresponds to or is derived from the Apelin protein naturally, i.e. without any recombinant intervention, encoded by the cell used in the methods of the invention. Alternatively, a cell used in the methods of the invention may encode an Apelin protein, fragment thereof or variant thereof which is heterologous to said cell. Preferably, the Apelin protein, fragment thereof or variant thereof corresponds to or is derived from human or murine Apelin and preferably corresponds to or is derived from an Apelin protein comprising an amino acid sequence according to SEQ ID NO:5 or 6 of the sequence listing. A fragment of Apelin preferably corresponds to amino acid residues 23 to 48, 27 to 41, or 65 to 77 of an Apelin protein, preferably of murine Apelin, more preferably of the sequence shown in SEQ ID NO:6 of the sequence listing, or corresponds to amino acid residues of an Apelin protein corresponding to the afore-mentioned amino acid residues in a homology comparison. In a particularly preferred embodiment, a fragment of Apelin has an amino acid sequence selected from the group consisting of SEQ ID NO:12 to 14 of the sequence listing.

In the methods according to all aspects of the invention, said determining of the level of said at least one peptide and/or protein preferably comprises a determination of the relative or absolute quantity of said at least one peptide and/or protein.

In a preferred embodiment of the methods of the invention, said determining of the level of said at least one peptide and/or protein is performed using mass spectrometric methods, optionally in combination with separation methods, wherein said separation methods are preferably selected from the group consisting of gel electrophoresis, liquid chromatography, gas chromatography, capillary chromatography, thin layer chromatography, mass spectrometry, precipitation techniques, liquid phase extraction techniques, filtration and other molecular size discriminating techniques.

The mass spectrometric methods are preferably selected from the group consisting of MALDI mass spectrometry, ESI mass spectrometry, SELDI mass spectrometry, FAB mass spectrometry and MS/MS mass spectrometry.

According to the invention, a thiazolidinedione is preferably selected from the group consisting ofRosiglitazone, Pioglitazone, Troglitazone, MCC-555, MK-0767, TZD18, Balaglitazone, Farglitazar, and Darglitazone and the disease is preferably selected from the group comprising Type II diabetes, impaired glucose tolerance, obesity, metabolic syndrome, cardiovascular diseases and atherosclerosis.

### Brief description of the drawings

Figure 1:

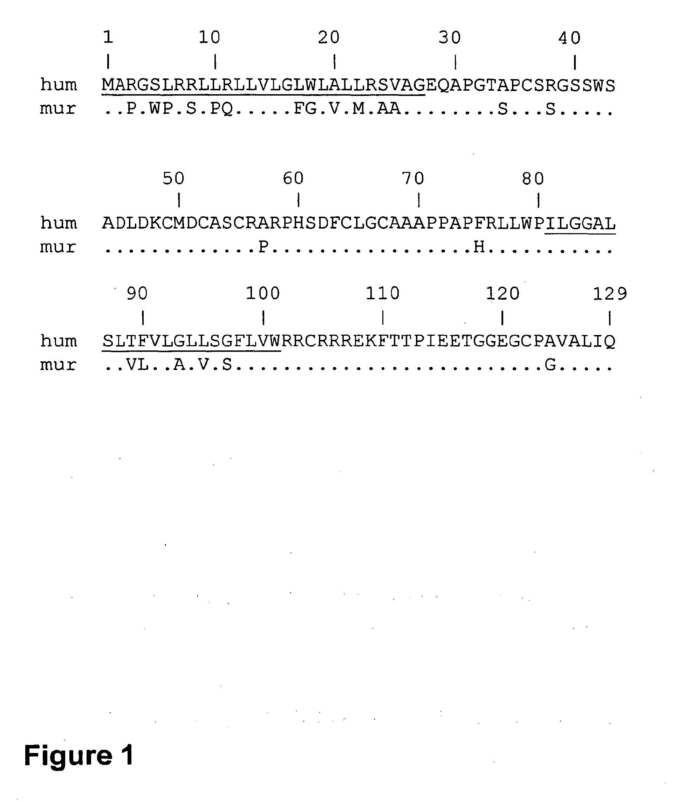

   Figure 1 shows an alignment of the human (hum) and murine (mur) sequences of Fnl4. Amino acid residues 1 to 27 of the human sequence are predicted to represent the signal sequence (underlined) and amino acid residues 81 to 101 are predicted to represent the transmembrane region (underlined). The N-terminus of Fn14 is located extracellular, whereas the C-terminus is located intracellular.
Figure 2:

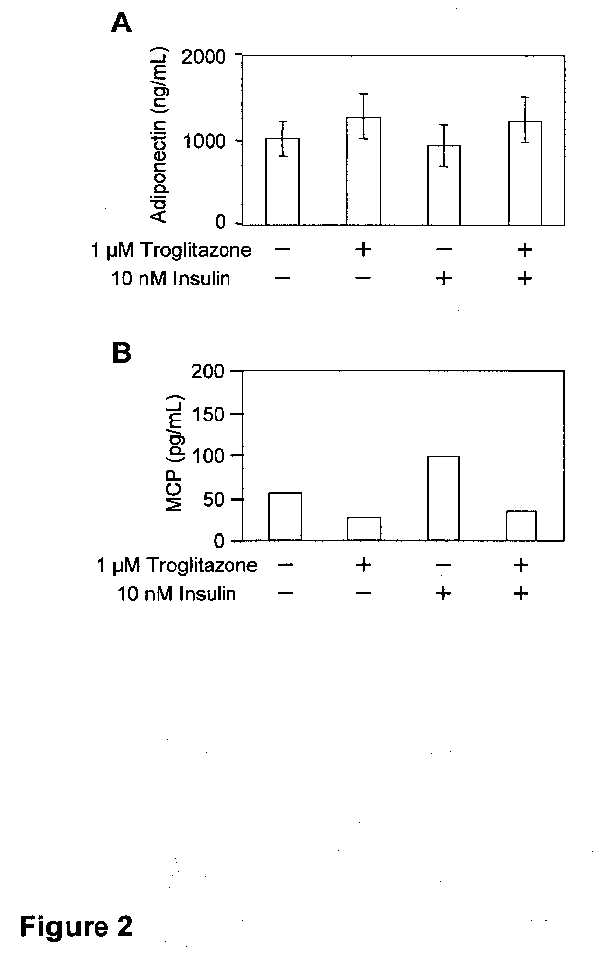

   Figure 2 shows the concentration of Adiponectin (panel A) and MCP-1 (panel B), as measured in cell culture supernatants of 3T3-L1 cells (3T3 cells differentiated into fat cells = 3T3-L1 cells). The cells were either not stimulated (left box plot) or stimulated for 40 hrs with 1 µM Troglitazone (first stimulus) followed by 8 hrs with 1 µM Troglitazone (a thiazolidinedione), 10 nM insulin, or both (right 3 box plots; second stimulus).
Figure 3:

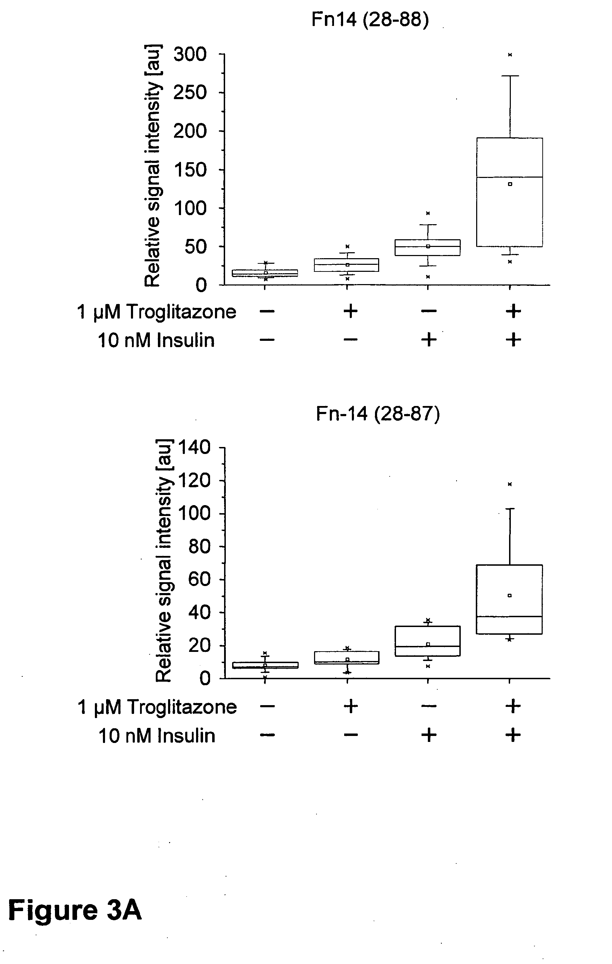

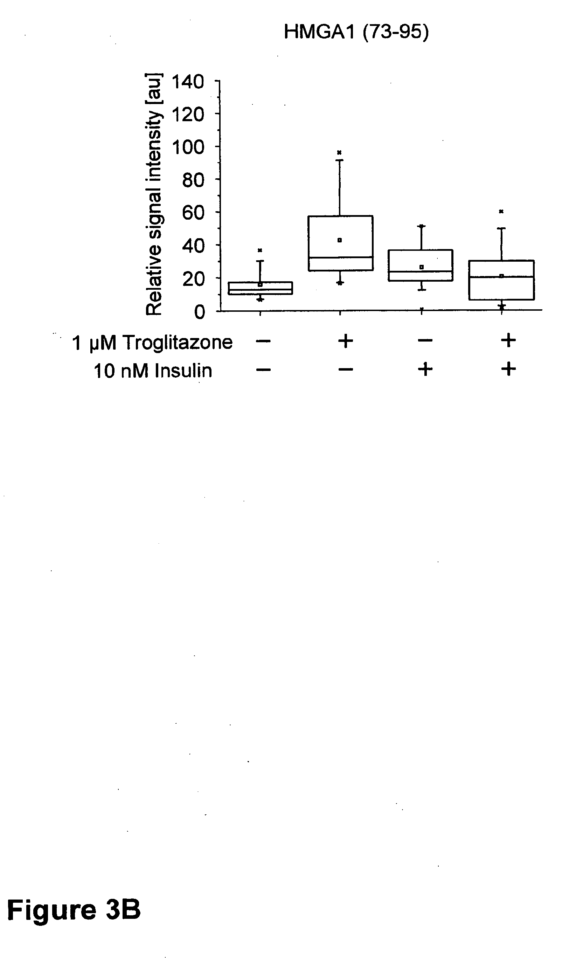

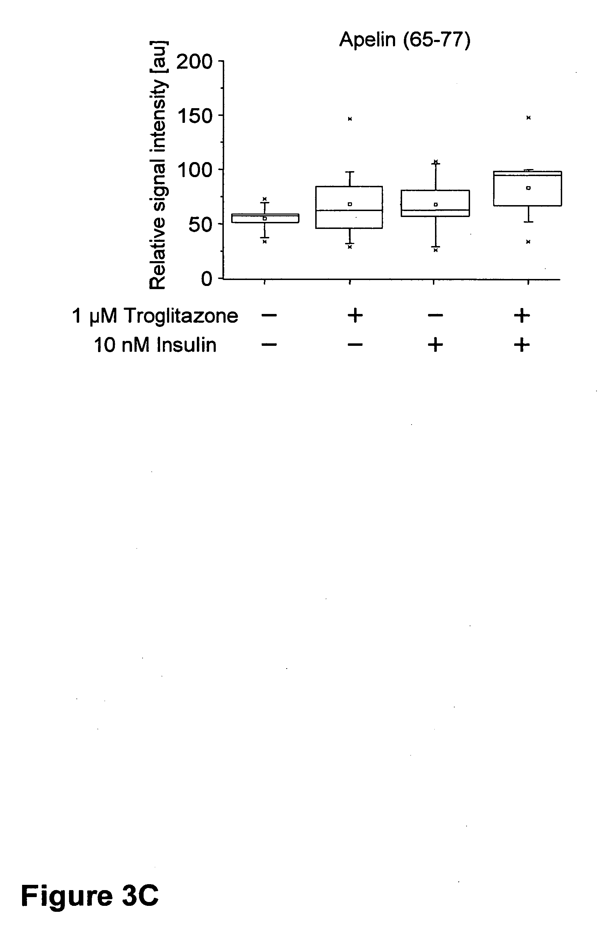

   Figure 3 shows the signal intensity of particular peptide fragments as determined in samples prepared according to example 3 from cell culture supernatants of 3T3-L1 cells (differentiated into fat cells), which were either not stimulated or stimulated for 40 h with 1µM Troglitazon alone, followed by a second stimulus for 8 h with 1 µM Troglitazone, 10 nM insulin or both. The samples were measured using mass spectrometry according to example 4. The y-axis represents the mass spectrometric signal intensity of the indicated peptides in samples of experimental groups, treated as indicated on the x-axis. A. Results for peptide fragments of Fn14, representing amino acid residues 28 to 88, and amino acid residues 28 to 87, respectively, B. Results for a peptide fragment of High Mobility Group A1 (HMGA1) representing amino acid residues 73 to 95, C. Results for a peptide fragment, representing amino acid residues 65-77 of Apelin.
Figure 4:

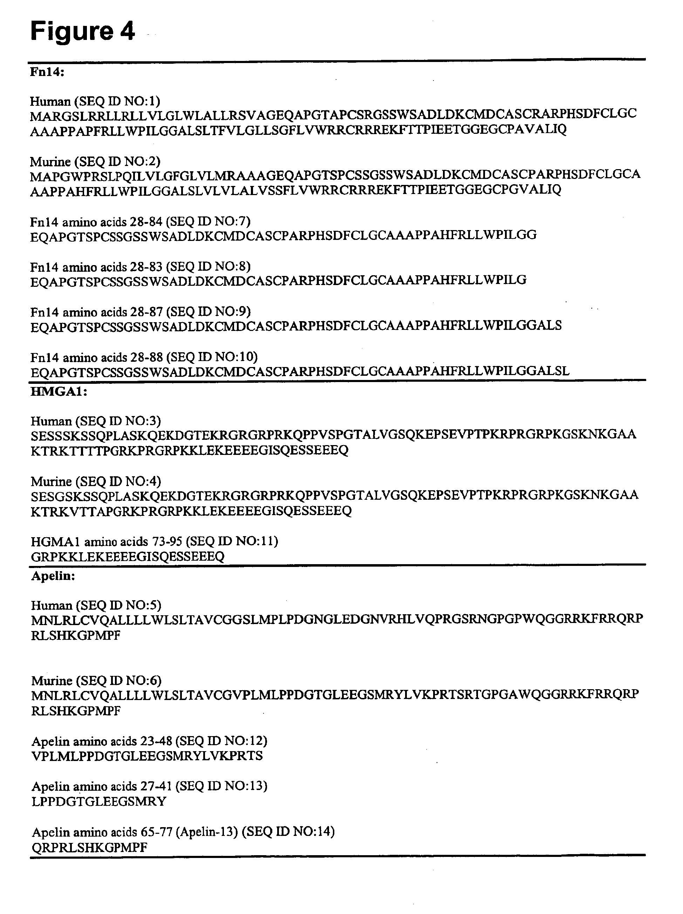

   Figure 4 shows sequences described herein.

### Detailed description of the invention

### Cells used in the methods of the invention

The term "cell" according to the invention relates to all kinds of cells including prokaryotic and eukaryotic cells, in particular bacterial cells, yeast cells, mammalian cells, rodent cells human cells, animal cells such as cells from mice, rats, hamsters, guinea pigs, gerbils, rabbits, zebra fish, drosophila flies, pigs, dogs, cats and apes. The cells may be derived from tissues such as visceral fat, abdominal fat or fat from other tissues, muscle, blood, intestine, pancreas, or liver tissue. For use in the methods of the invention, the cells may be present within a living organism and/or within a tissue, and may be cultured cells and cell lines.

Preferred cells used according to the invention are cells involved in blood glucose level regulation and/or cells involved in Type II diabetes pathology. Preferably, the cells express a PPAR-receptor, in particular PPAR-gamma and/or are responsive to treatment with a thiazolidinedione. Particularly preferred cells used according to the invention are fat cells, in particular adipocytes.

In certain embodiments of the present invention, the cell may be a cell modified using molecular biologic methods. For example, for testing the effect of a candidate substance on the level of at least one peptide and/or protein selected from the group consisting of Fn14, HMGA1, Apelin, fragments thereof, and variants thereof, a nucleic acid coding for a peptide or protein selected from the afore mentioned group may be introduced into a cell or cell membrane which does not naturally express or contain said peptide or protein encoded by said nucleic acid, in particular a cell that does not naturally express Fn14, HMGA1 and Apelin, respectively. Alternatively, a further copy or multiple copies of said nucleic acid may be introduced into a cell already containing said nucleic acid to increase the signal obtained in the methods of the present invention.

### Contacting a cell with a candidate substance

The expression "contacting a cell with a candidate substance" refers to the situation that at least one cell which may be present in a sample in vitro or in vivo within a living organism and the candidate substance come into contact.

The term "candidate substance" according to the invention refers to any substance which can be tested in the methods of the invention, wherein the term substance comprises compounds and compositions.

The term "compound" according to the invention relates to all kinds of molecules including organic molecules (containing carbon), preferably small organic molecules, or inorganic molecules (not containing carbon), chemically, enzymatically or recombinantly synthesized molecules, such as nucleic acid sequences, modified nucleic acid sequences, amino acid sequences, modified amino acid sequences, peptides, proteins, etc. as well as derivatives thereof, synthetic molecules or molecules found in nature or molecules isolated from materials or organisms found in nature, for example found in extracts of plants, yeast, animals or microorganisms, as well as molecules found in nature with altered structures or with sequences comprising deletions, insertions and mutations of the sequence and combinations thereof. Preferably the small molecule has a molecular weight below 2000 Dalton, more preferably below 1000 Dalton.

Compositions according to the invention may comprise two or more compounds to be tested, in particular a library of compounds, or may comprise at least one compound to be tested and additional components such as saline, diluents, solvents, pH buffering agents, osmolarity or viscosity regulating agents, additives for preservation, staining, flavouring or scenting, filling substances, anti-microbial, anti-fungal or other preserving agents, substances protecting the compound or composition from light, in particular daylight, UV light, heat, freezing, or other environmental factors, components preventing aggregation or sticking of compounds to surfaces or containers, formulations necessary for specific routes of applications such as gastric acid resistant capsules, or plasters for transdermale application, sterile or isotonic solutions for intravenous, subcutan, intradermal, etc. injections, or for infusions, or formulations suitable for generating aerosols for inhalation, etc. Furthermore, components preventing proteolysis or other forms of degradation or modification of a constituent of the composition may be present in a composition.

The expression "library of compounds" refers to a multitude of compounds which can be obtained by purification from natural sources such as microorganisms, plants, yeasts, animals, etc., by molecular biologic strategies obtaining a multitude of gene products, by random or directed synthesis of nucleic acid or amino acid sequences, by combinatorial chemistry, by rational drug design, etc.

The expression "candidate" with respect to a substance refers to any substance, which is useful for testing in the methods of the invention. In general, if a candidate substance is tested for its ability to alter the level of peptides and/or proteins in the methods of the invention, it will be unknown before such testing whether the candidate substance tested has such ability or not. However, in other embodiments there may be indications or information on whether a candidate substance to be tested has such ability or not. This includes, for example, situations where a candidate substance has already been tested in assays different to that of the present invention and the candidate substance is tested by the methods of the invention to confirm the results obtained in said different assays, obtain quantitative information about the activity of the candidate substance, test the candidate substance in a different system and/or environment and/or compare the candidate substance with another substance and/or compare the candidate substance in different concentrations and/or for different periods of time and/or administrated by different means. Indications or information on whether a candidate substance to be tested has the ability to alter the level of peptides and/or proteins in the methods of the invention or not may also exist, for example, if the structure or chemical composition of the substance being tested is known and is similar or related to the structure or composition of a different substance which is known or suspected to have the above-mentioned ability or not.

### Determining the level of at least one peptide and/or protein

The expression "determining the level of at least one peptide and/or protein" according to the invention relates to the determination of the absence or presence and/or the absolute and/or relative quantification of said at least one peptide and/or protein. The expression "at least one peptide and/or protein selected from the group consisting of Fn14, HMGA1, and Apelin, fragments thereof, and variants thereof" relates to a single member of said group or a combination of at least 2, preferably at least 3, 4, 5, 6, 7, 8, 9, or 10 or more members of said group. The expression "determining the level of at least one peptide and/or protein" according to the invention also includes situations wherein no peptide and/or protein is detected or the amount of said peptide and/or protein is below the detection limit which situations may be indicative for the fact that a candidate substance does not increase the level of said peptide and/or protein or reduces the level of said peptide and/or protein below the detection limit.

### Thiazolidinediones

The term "thiazolidinedione" according to the invention includes but is not limited to Rosiglitazone, Pioglitazone, Troglitazone, MCC-555, MK-0767, TZD18, Balaglitazone, Farglitazar, Ciglitazone, Darglitazone, LSN862, PAT5A, FK614. Troglitazones and other "glitazones" belong to the chemical class of thiazolidine-2,4-diones. Novel agents share a substituted thiazolidinedione structure with modifications to improve the pharmacological efficacy. Based on structure activity relationship (SAR) several novel non-thiazolidinedione PPAR agonists were developed having improved anti-diabetic activity including muraglitazar/BMS-298585, RWJ-348260, nTZDpa.

### Substance having at least one functional property of a thiazolidinedione

The expression "at least one functional property of a thiazolidinedione" according to the invention relates to all functional activities of thiazolidinediones. Such functional activities comprise the effect on the level of at least one peptide and/or protein selected from the group consisting of Fn14, HMGA1, and Apelin, fragments thereof, and variants thereof, or other biomarkers for thiazolidinediones, such as proteins the regulation of which is regulated by PPAR, in particular PPAR-gamma, a binding to PPAR, in particular PPAR-gamma, an improvement in insulin resistance, an increase in glucose uptake by muscle cells, an inhibition of lipolysis or hepatic gluconeogenesis, etc. Further examples of functional activities of thiazolidinediones are anti-diabetic, anti-Type II diabetic, anti-obesity, anti-metabolic syndrome, anti-impaired glucose tolerance, anti-atherosclerosis and anti-coronary heart disease activities. If a substance has a functional property of a thiazolidinedione, the level of exhibiting this functional property may be similar or different to the thiazolidinedione, i.e. comparable, increased or decreased.

### Substance having analogous activity to a thiazolidinedione

The expression "substance having analogous activity to a thiazolidinedione" according to the invention relates to a substance which may serve as a substitute for a thiazolidinedione, in particular in therapy, specifically in the therapy of Type II diabetes, obesity, impaired glucose tolerance, metabolic syndrome (syndrome x), cardiovascular diseases, atherosclerosis and/or dyslipidemia. In this case, the substance having analogous activity to a thiazolidinedione will normally have several functional properties of a thiazolidinedione and preferably, will have all functional properties of a thiazolidinedione, preferably at the same or a comparable level.

### Disease treatable with a thiazolidinedione /disease associated with a PPAR-gamma receptor

The expression "disease treatable with a thiazolidinedione" relates to any pathologic condition which can be treated with a thiazolidinedione and includes but is not limited to Type II diabetes, insulin resistance, obesity, impaired glucose tolerance, metabolic syndrome (syndrome x), cardiovascular diseases, atherosclerosis and dyslipidemia. The expression "disease associated with a PPAR-gamma receptor" includes diseases treatable with ligands of PPAR-gamma such as thiazolidinediones, including but not limited to the afore-mentioned diseases.

The term "treatment of a disease" includes curing, shortening the duration, ameliorating, preventing, slowing down or inhibiting progression or worsening, or preventing or delaying the onset of a disease or the symptoms thereof. "Therapeutic agent" or "therapeutic" relates to substances having a therapeutic effect and being useful in the therapy or treatment of a disease.

### Peptides, proteins, fragments thereof and variants thereof

The term "peptide" refers to substances comprising two or more, preferably 3 or more, preferably 4 or more, preferably 6 or more, preferably 8 or more, preferably 10 or more, preferably 13 or more, preferably 16 or more, preferably 21 or more and up to preferably 50, in particular 60 or in particular 70 consecutive amino acids joined covalently by peptide bonds. The term "protein" refers to large peptides, preferably to peptides with at least 71 amino acid residues, but in general the terms "peptides" and "proteins" are synonyms and are used in this application as synonyms. The terms "peptide" and "protein" according to the invention also include substances containing not only amino acid residues, but also non-amino acid constituents such as sugar or phosphate structures and includes also substances containing only peptide bonds as well as substances containing other bonds, e.g. ester, thioether or disulfide bonds.

The term "fragment of a protein" relates to a peptide or protein which has a deletion of one or more amino acid residues compared to the full-length protein. In particular, a "fragment of a protein" has a length sufficient to unequivocally identify the protein from which it is derived.

In preferred embodiments, a fragment of a protein has a sequence of 4 or more, preferably 6 or more, preferably 8 or more, preferably 10 or more, preferably 15 or more, preferably 20 or more, preferably 40 or more and up to preferably 100, in particular 80, 60, 50, 40, 30, or 20 consecutive amino acids.

A "variant" of a peptide or protein or a "variant of a sequence" as used herein, comprises a peptide or protein or sequence thereof that differs from a peptide or protein from which it is derived in one or more substitutions, deletions, additions, insertions and/or modifications of amino acids. The terms "variant" and "derivative" are used as synonyms herein.

Preferably, a variant of a peptide or protein has a functional property of the peptide or protein from which it is derived. Thus, a variant may exhibit substantially the same structure, function, antigenicity, immunogenicity etc., as the peptide or protein from which it is derived.

Peptide or protein variants according to the invention include those exhibiting at least about 80%, 85%, 90%, 91%, 92%, 93%, 94%, 95%, 96%, 97%, 98%, or 99% or more identity to the peptide or protein sequences from which they are derived.

The term "percentage identity" is intended to denote a percentage of nucleotides or of amino acid residues which are identical between the two sequences to be compared, obtained after the best alignment, this percentage being purely statistical and the differences between the two sequences being distributed randomly and over their entire length. Sequence comparisons between two nucleotide or amino acid sequences are conventionally carried out by comparing these sequences after having aligned them optimally, said comparison being carried out by segment or by "window of comparison" in order to identify and compare local regions of sequence similarity. The optimal alignment of the sequences for comparison may be produced, besides manually, by means of the local homology algorithm of Smith and Waterman, 1981, Ads App. Math. 2, 482, by means of the local homology algorithm of Neddleman and Wunsch, 1970, J. Mol. Biol. 48, 443, by means of the similarity search method of Pearson and Lipman, 1988, Proc. Natl Acad. Sci. USA 85, 2444, or by means of computer programs which use these algorithms (GAP, BESTFIT, FASTA, BLAST P, BLAST N and TFASTA in Wisconsin Genetics Software Package, Genetics Computer Group, 575 Science Drive, Madison, Wis.).

The percentage identity between two nucleic acid or amino acid sequences is determined by comparing these two sequences aligned in an optimal manner in which the nucleic acid or amino acid sequence to be compared may comprise additions or deletions compared to the reference sequence for optimal alignment between these two sequences. The percentage identity is calculated by determining the number of identical positions for which the nucleotide or the amino acid residue is identical between the two sequences, dividing this number by the number of positions compared and multiplying the result obtained by 100 so as to obtain the percentage identity between these two sequences.

Preferably, a variant contains conservative substitutions. A "conservative substitution" is one in which an amino acid is substituted for another amino acid that has similar properties, such that one skilled in the art of peptide chemistry would expect the characteristics such as secondary structure, charge, and hydropathic nature of the peptide or protein to be substantially unchanged. Amino acid substitutions may generally be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his. A variant may also, or alternatively, contain non-conservative changes.

The peptides or proteins described herein may also comprise any natural and non-natural modifications, in particular chemical or physical modifications. The term "natural modification" relates to any modification found in peptides or proteins in nature such as posttranslational modifications, modifications due to exposure to light, oxygen, or acid or alkali solutions, chemical modifications, enzymatical modifications etc.

Examples of possible modifications include but are not limited to: glycosylation, phosphorylation, sulphatation, pyroglutamate modification, cystein-disulfide bridges, methylation, acetylation, acylation, farnesylation, formylation, geranylgeranylation, biotinylation, stearoylation, palmitylation, lipolyation, C-mannosylation, myristoylation, amidation, deamidation, methylation, demethylation, carboxylation, hydroxylation, iodination, oxidation, pegylation, prenylation, ADP-ribosylation, addition of lipids, of phosphatidylinositol, of glycosylphosphatidylinositol (GPI)-anchor, of pyridoxal phosphate, modification of cysteine residues resulting in carboxyamidomethylcysteine, resulting in carboxymethylcysteine, or resulting in pyridylethylcysteine, modification of lysine residues resulting in liponic acid, modification of glutamic acid resulting in pyroglutamic acid, etc.

Modifications of peptides or proteins described herein may comprise unusual amino acids, chemically or enzymatically modified amino acids etc. including, but not limited to: alpha amino butyric acid, beta amino butyric acid, beta amino iso-butyric acid, beta alanine, gamma butyric acid, alpha amino adipic acid, 4-amino benzoic acid, amino ethyl cysteine, alpha amino penicillanic acid, allysine, 4-carboxy glutamic acid, cystathionine, carboxy glutamic acid, carboxy amido methyl cysteine, carboxy methyl cysteine, cysteine acid, citrulline, dehydroalanine, di-amino butyric acid, dehydro amino-2-butyric acid, ethionine, glycine-proline di-peptide, 4-hydroxyproline, hydroxylysine, hydroxyproline, homoserine, homo cysteine, histamine, iso-valine, lysinoalanine, lanthionine, norvaline, norleucine, ornithine, 2-pipiridine-carboxylic acid, pyroglutamic acid, pyrrolysine, proline-hydroxy proline di-peptide, sarcosine, 4-selenocysteine, syndesine, thioproline, etc.

The peptides or proteins described herein may be modified to facilitate their detection, in particular by labelling, or alter their stability during storage or usage.

With "secretion of peptides and/or proteins" are meant all kinds of mechanisms leading to release of peptides and/or proteins from the intracellular region of a cell or from the surface of the cell membrane (e.g. by proteolytic release of the extracellular domain of membrane proteins and/or peptides = shedding). Examples of mechanisms of release of peptides or proteins are (i) the protein translocation across the cell membrane secretion via the classical secretory pathway, (ii) the protein translocation across the cell membrane secretion via the non-classical secretory pathway (proteins or peptides lacking a classical N-terminal signal peptide, such as interleukin 1 beta, fibroblast growth factor 1 and 2, FGF-1 and FGF-2, galectins), (iii) membrane proteins having extracellular domains that are subject to proteolytic release, a process known as shedding (TNF-alpha, amyloid precursor protein, TNF-alpha receptor) and (iv) regulated intramembrane proteolysis (Rip) a process requiring the activity of enzymes such as gamma-secretase and of cofactors (amyloid precursor protein, CD44).

### Labels

According to the invention, molecules may be labelled by one or more labels for detection. If more than one label is present, the labels may be different or the same, may be of the same or a different class, may be at different or the same positions within the labelled molecule, etc.

The term "label", as used herein, refers to any moiety that functions to: (i) provide a detectable signal; (ii) interact with a second label to modify the detectable signal provided by the first or second label, e.g. FRET (Fluorescence Resonance Energy Transfer); (iii) affect mobility, e.g. electrophoretic mobility, by charge, hydrophobicity, shape, or other physical parameters, or (iv) provide a capture moiety, e.g., affinity, antibody/antigen, or ionic complexation.

Suitable as label are structures of various classes of labels, such as stable or radioactive isotopic labels, isobaric labels, fluorophore labels, fluorescent proteins as labels, luminescent labels, enzyme labels, toxin labels, dye labels, metal, magnetic, or polymer particles as labels, biotin, chitin, maltose, glutathione etc. or other organic or inorganic molecules.

Preferably isotopes with molecular weights different to their natural molecular weight such as oxygen-18, nitrogen-15, deuterium, tritium, carbon-14, sulphur-35, phosphorus-32, phosphorus-33 etc. can be used to prepare isotope-labelled molecules, especially as other isotopes of these elements are naturally building blocks of molecules such as proteins, peptides, nucleic acid sequences etc. The isotopes can be incorporated directly into the molecule, for example, in the case of peptides or proteins by using isotope-labelled amino acids for synthesis of the peptides or proteins. Furthermore, isotopes can be incorporated into, for example, peptides or proteins by growing cells in the presence of isotope-labelled metabolites and isolating the labelled peptides or proteins from these cells or cell culture supernatants. The isotopes can also be incorporated into molecules such as peptides or proteins by other methods such as in vitro translation using isotope-labelled metabolites. Isotope-labelled metabolites can be amino acids, carbohydrates, fatty acids, inorganic salts such as phosphate, sulphate, etc. or other isotope-labelled substances. Isotope-labelled peptides or proteins are especially suitable for measuring methods using mass spectrometric methods.

Another kind of label especially suitable for mass spectrometric detection methods are isobaric labels. Different kinds of such labels have the same molecular weight, as long as these labels are intact and have not been fragmented in a mass spectrometer. This has the advantage that mixtures of molecules labelled with different isobaric labels can more conveniently be analyzed using mass spectrometric methods, as only a limited number of mass differences is present prior to analysis of the sample. Fragmentation of the isobaric labels may be done to determine the identity of the label, thereby identifying the identity of the molecule labelled with the isobaric label. Preferably these labels are constructed in a way that, upon fragmentation of the label in a mass spectrometer, the fragments generated have masses, which masses usually are not obtained by fragmentation of unlabelled molecules such as peptides or proteins. Examples of such masses are masses between 114 and 120 Dalton. Preferably isobaric labels are used, which upon fragmentation do no result in masses, which are also generated in the same kind of samples, analyzed by the same mass spectrometric method in the absence of said isobaric labels.

Examples of radioactive isotopes, which may be used as labels include iodine-125, calcium-45, chromium-51, tritium, carbon-14, sulphur-35, phosphorus-32, phosphorus-33, etc. Furthermore, all other known radioisotopes or stable isotopes which can be coupled to nucleic acid sequences and/or amino acid sequences may be used as labels.

Examples of fluorescent labels are fluorophores such as Alexa Fluor® 350, 405, 430,488, 500, 514, 532, 546, 555, 568, 594, 610, 633, 635, 647, 660, 680, 700 and Alexa Fluor® 750, Aminomethylcoumarin (AMCA), Methoxy coumarin acetic acid, Bimane, BODIPY 493/503, 530/550, 558/568,564/570,576/589, 581/591, 630/650 and BODIPY 650/665, BODIPY FL, BODIPY TR; BODIPY TMR, Cascade Blue dye, Cascade Yellow dye, cyanine dyes such as Cy3, Cy5, Cy5.5, Cy7, Dansyl, Dapoxyl dye, Dialkylaminocoumarin, Eosin, Erythrosin, Fluorescein (FITC), Fluorescein-EX, 2',4',5',7'-Tetrabromosulfonefluorescein, Naphthofluorescein, 2',7'-Dichloro-fluorescein, 4',5'-Dichloro-2',7'-dimethoxy-fluorescein, 6-Carboxy-2',4,4',5',7,7'-hexachlorofluorescein succinimidyl ester (6-HEX, SE), 5-carboxyfluorescein (5FAM), 5,6-carboxyfluorescein (5(6)FAM), hydroxycoumarin, Malachite green, Marina Blue dye, methoxycoumarin, 7-nitro-4-benzofurazanyl (NBD), Oregon Green® 488, Oregon Green® 514, Pacific Blue, Pacific Blue dye, PyMPO, Pyrene, QSY 7 , QSY 9, QSY 21, QSY 35, Rhodamine 6G, Rhodamine Green, Rhodamine Green dye, Rhodamine Red dye, Rhodamine Red, Rhodamine Red-X (RRX), X-rhodamine, Tetramethyl-rhodamine (TMR, TRITC), Lissamine rhodamine B, Texas Red, Texas Red dye, Texas Red-X. Most of these fluorophores are available from Molecular Probes, Eugene, OR, USA, and many are also available as kits intended for protein or peptide labelling. Furthermore, fluorescent proteins or fragments or derivatives thereof such as green, red, blue, or renilla fluorescent protein, etc. may be used as labels. In another embodiment it is possible to use matched pairs of fluorescent labels, which are suitable for fluorescence resonance energy transfer (FRET).

Examples of enzyme labels are horse-raddish-peroxidase, alkaline phosphatase, luciferase, galactosidase, etc. Suitable toxin labels are all kinds of toxins such as microbial toxins, toxic peptides or toxic small organic molecules, synthetic toxins or toxins used for medical purposes. An example of suitable dye labels is digoxigenin.

Examples of particles as labels are particles of various particle diameters, preferably between 0.1 to 100 µm. Metal particles may be made from various substances, preferably heavy elements such as gold, silver, platinum or other suitable metals or alloys or mixtures thereof. Examples of magnetic particles are preferably made from all kinds of magnetic or magnetizable materials including iron, preferably iron oxides such as FeO, Fe₂O₃, Fe₃O₄, etc. Examples of polymer particles are particles made from a polymer such as polyacrylamide, agarose, polypropylene, polystyrene, polytetrafluoroethylene, fluorescent polystyrene microspheres, etc. Various other labels, such as biotin, chitin, maltose or glutathione etc. as known in the art, may also be used.

Labelled amine-reactive reagents such as isothiocyanates, succinimidyl esters and carboxylic acids, sulfonyl chlorides, aldehydes, arylating reagents, thiol-reactive reagents such as iodoacetamides, maleimides, alkylhalides and arylating agents, may be used to couple labels to molecules. Furthermore, alcohol moieties such as those present in serine, threonine or in carbohydrate moieties may be labelled by oxidizing using periodate to yield aldehydes that can be subsequently modified with a variety of amine or hydrazine derivatives. Alcohol moieties of tyrosine sometimes may be reacted with sulfonyl chlorides or iodoacetamides. Alcohol moieties in carbohydrates furthermore may be labelled by reaction with dichlorotriazines. N-methylisatoic anhydride may be used to convert ribonucleotides and other carbohydrate moieties to fluorescent esters with excitation/emission maxima of about 350/466 nm.

Furthermore, labels comprising certain nucleic acid or amino acid sequences subsequently termed label-sequences can be used. Label-sequences comprising nucleic acid sequences for example can be used for detection of the labelled substance for example by hybridization with a probe specific for the label-sequence or by detection of the label-sequence by polymerase chain reaction, preferably by quantitative polymerase chain reaction, preferably by real-time polymerase chain reaction, etc. Examples of label-sequences comprising amino acid sequences are his-tags, flag-tags, myc-tags or whole proteins or fragments thereof such as antibodies, glutathione S-transferase, streptavidin, chitin-binding protein, maltose binding protein, various lectins, etc. These labels can be detected by use of antibodies or by use of ligands binding to these labels such as protein A, protein G, protein Y, protein A/G, glutathione, biotin, chitin, maltose or other sugars etc. Label-sequences in general can also be used to capture the labelled peptide or protein by a binding agent specific for said label-sequence. Subsequently or during the capture process the labelled peptide or protein can be quantified. Label-sequences can be at the N- or C-terminus or can be present internally in an amino acid sequence or can be at the 3'- or 5'-end or internally in a nucleic acid sequence.

### Antibodies

The term "antibody" includes antibodies of any isotype (e.g., IgG, IgA, IgM, IgE, IgY, etc.), and includes fragments of antibodies binding to the same epitope as the antibody from which it is derived. Fragments of antibodies may be prepared recombinantly or by proteolytic cleavage of an antibody. Non-limiting examples of such fragments include Fab, F(ab')₂, Fab', Fv, and single chain antibodies (scFv) containing at least one V[L] and/or V[H] domain joined with or without a linker such as a peptide linker. The scFv may be covalently or non-covalently linked to form antibodies having two or more binding sites. Furthermore included by the term "antibody" are polyclonal, oligoclonal, monoclonal, humanized, or recombinant antibodies. Antibodies may be present in a purified state or in the form of an anti-serum, in the form of ascites, as cell culture supernatants, in lyophilized form, or coupled to surfaces of for example ELISA plates, chips, membranes, or to the surface of agarose, metal or other beads or particles, etc.

### Specific binding

According to the invention, the term "binding" preferably relates to a specific binding. "Specific binding" means that an agent such as an antibody binds stronger to a target such as an epitope for which it is specific compared to the binding to another target. An agent binds stronger to a first target compared to a second target if it binds to the first target with a dissociation constant (K_{D}) which is lower than the dissociation constant for the second target. Preferably the dissociation constant (K_{D}) for the target to which the agent binds specifically is more than 10-fold, preferably more than 20-fold, more preferably more than 50-fold, even more preferably more than 100-fold, 200-fold, 500-fold or 1000-fold lower than the dissociation constant (K_{D}) for the target to which the agent does not bind specifically.

### Living organism

The expression "living organism" refers to all kinds of multicellular organisms including plants, animals, preferably mammals and includes humans, mice, rats, hamsters, guinea pigs, gerbils, rabbits, zebra fish, drosophila flies, pigs, dogs, cats, apes etc. The expression "individual" is used interchangeably with the term "living organism".

### Nucleic acids

According to the invention, a nucleic acid is preferably deoxyribonucleic acid (DNA) or ribonucleic acid (RNA). Nucleic acids comprise according to the invention genomic DNA, cDNA, mRNA, recombinantly produced and chemically synthesized molecules. According to the invention, a nucleic acid may be present as a single-stranded or double-stranded and linear or covalently circularly closed molecule.

"Variants" of a nucleic acid means according to the invention that single or multiple nucleotide substitutions, deletions and/or additions are present in said nucleic acid. Preferably, a "variant" of a nucleic acid is complementary to said nucleic acid. Furthermore, the term "variant" also comprises derivatization of a nucleic acid on a nucleotide base, on the sugar or on the phosphate. The term "variant" also comprises nucleic acids which contain nucleotides and nucleotide analogs not occurring naturally.

The nucleic acids described according to the invention have preferably been isolated. The term "isolated nucleic acid" means according to the invention that the nucleic acid was (i) amplified *in vitro,* for example by polymerase chain reaction (PCR), (ii) recombinantly produced by cloning, (iii) purified, for example by cleavage and gel-electrophoretic fractionation, or (iv) synthesized, for example by chemical synthesis. An isolated nucleic acid is a nucleic acid which is available for manipulation by recombinant DNA techniques.

A nucleic acid is "complementary" to another nucleic acid if the two sequences are capable of hybridizing and forming a stable duplex with one another, with hybridization preferably being carried out under conditions which allow specific hybridization between polynucleotides (stringent conditions). Stringent conditions are described, for example, in Molecular Cloning: A Laboratory Manual, J. Sambrook et al., Editors, 2nd Edition, Cold Spring Harbor Laboratory press, Cold Spring Harbor, New York, 1989 or Current Protocols in Molecular Biology, F.M. Ausubel et al., Editors, John Wiley & Sons, Inc., New York and refer, for example, to hybridization at 65°C in hybridization buffer (3.5 x SSC, 0.02% Ficoll, 0.02% polyvinylpyrrolidone, 0.02% bovine serum albumin, 2.5 mM NaH₂PO₄ (pH 7), 0.5% SDS, 2 mM EDTA). SSC is 0.15 M sodium chloride/0.15 M sodium citrate, pH 7. After hybridization, the membrane to which the DNA has been transferred is washed, for example, in 2 × SSC at room temperature and then in 0.1-0.5 × SSC/0.1 × SDS at temperatures of up to 68°C.

According to the invention, complementary nucleic acids have at least 40%, in particular at least 50%, at least 60%, at least 70%, at least 80%, at least 90% and preferably at least 95%, at least 98% or at least 99%, identical nucleotides.

Nucleic acids may, according to the invention, be present alone or in combination with other nucleic acids, in particular heterologous nucleic acids, and may be functionally linked to said other nucleic acids. In preferred embodiments, a nucleic acid is functionally linked to expression control sequences which may be homologous or heterologous with respect to said nucleic acid. A coding sequence and an expression control sequence are "functionally" linked to one another, if they are covalently linked to one another in such a way that expression or transcription of said coding sequence is under the control or under the influence of said expression control sequence. If the coding sequence is to be translated into a functional protein, then, with an expression control sequence functionally linked to said coding sequence, induction of said expression control sequence results in transcription of said coding sequence, without causing a frame shift in the coding sequence or said coding sequence not being capable of being translated into the desired protein or peptide.

The term "expression control sequence" comprises according to the invention promoters, enhancers and other control elements which regulate expression of a gene. In particular embodiments of the invention, the expression control sequences can be regulated. The exact structure of regulatory sequences may vary as a function of the species or cell type, but generally comprises 5'untranscribed and 5'untranslated sequences which are involved in initiation of transcription and translation, respectively, such as TATA box, capping sequence, CAAT sequence, and the like. More specifically, 5'untranscribed regulatory sequences comprise a promoter region which includes a promoter sequence for transcriptional control of the functionally linked gene. Expression control sequences may also comprise enhancer sequences or upstream activator sequences.

According to the invention, a nucleic acid may furthermore be present in combination with another nucleic acid which represents a reporter gene or any "tag".

In a preferred embodiment, a recombinant nucleic acid molecule according to the invention is a vector, where appropriate with a promoter, which controls expression of a nucleic acid. The term "vector" is used here in its most general meaning and comprises any intermediary vehicle for a nucleic acid which enables said nucleic acid, for example, to be introduced into prokaryotic and/or eukaryotic cells and, where appropriate, to be integrated into a genome. Vectors of this kind are preferably replicated and/or expressed in the cells. An intermediary vehicle may be adapted, for example, to the use in electroporation, in bombardment with microprojectiles, in liposomal administration, in the transfer with the aid of agrobacteria or in insertion via DNA or RNA viruses. Vectors comprise plasmids, phagemids, bacteriophages or viral genomes.

The nucleic acids described according to the invention may be used for transfection of host cells. Nucleic acids here mean both recombinant DNA and RNA. Recombinant RNA may be prepared by in-vitro transcription of a DNA template. Furthermore, it may be modified by stabilizing sequences, capping and polyadenylation prior to application. According to the invention, the term "host cell" relates to any cell which can be transformed or transfected with an exogenous nucleic acid. Host cells include prokaryotic and eukaryotic cells, such as bacteria, yeast cells, plant cells, animal cells, mammalian cells, in particular human cells, cell lines, primary cells, stem cells, etc.

According to the invention, the term "expression" is used in its most general meaning and comprises the production of RNA or of RNA and peptide or protein. It also comprises partial expression of nucleic acids. Furthermore, expression may be carried out transiently or stably.

Preferred expression systems in mammalian cells comprise pcDNA3.1 and pRc/CMV (Invitrogen, Carlsbad, CA), which contain a selectable marker such as a gene imparting resistance to G418 (and thus enabling stably transfected cell lines to be selected) and the enhancer-promoter sequences of cytomegalovirus (CMV).

### Responsiveness to treatment

The expression "responsive to treatment" relates to the reaction of a cell or a living organism in response to a particular treatment, such as the administration of a particular substance, compound or composition. The reaction of a cell, in particular, concerns an alteration of the biochemistry of said cell such as an alteration in the level of particular peptides or proteins such as Fn14, HMGA1, Apelin, a fragment or variant thereof. The reaction of a living organism, in particular, concerns a benefit of the living organism from the treatment. If a living organism, for example, is responsive to treatment with a substance having at least one functional property of a thazolidinedione said substance preferably is effective in the treatment of the diseases treatable with thiazolidinediones as described above.

### Administration

According to the invention substances may be administered via any conventional route, including by injection or infusion. The administration may be carried out, for example, orally, intravenously, intraperitonealy, intramuscularly, subcutaneously, sublingual, transdermally or by inhalation of a gas or aerosol.

The substances are preferably administered in effective amounts. An "effective amount" refers to the amount which achieves a desired reaction or a desired effect alone or together with further doses or further medical or non-medical treatments. Non-medical treatments for example are physical exercises, special diets such as diets to prevent body mass gain, or to loose body mass, or diets to limit or increase intake of certain nutrients or food ingredients, such as for example sugars, fibers, antioxidants such as vitamin C, etc. A desired reaction includes, for examples, the treatment of a disease.

An effective amount of a substance will depend on the condition to be treated, the severeness of the disease, the individual parameters of the patient, including age, physiological condition, size and weight, the duration of treatment, the type of an accompanying therapy (if present), the specific route of administration and similar factors.

The doses of the substances administered may depend on various parameters such as the type of administration, the condition of the patient, the desired period of administration, etc. In the case that a reaction in a patient is insufficient with an initial dose, higher doses (or effectively higher doses achieved by a different, more localized route of administration) may be used.

The substances administered are preferably sterile. Furthermore, the substances are generally administered in pharmaceutically compatible amounts and in pharmaceutically compatible compositions. The term "pharmaceutically compatible" refers to a nontoxic material which does not interact with the action of the active component. Preparations of this kind may usually contain salts, buffer substances, preservatives, carriers and, where appropriate, other therapeutically active compounds. When used in medicine, the salts should be pharmaceutically compatible. However, salts which are not pharmaceutically compatible may used for preparing pharmaceutically compatible salts and are included in the invention. Pharmacologically and pharmaceutically compatible salts of this kind comprise in a nonlimiting way those prepared from the following acids: hydrochloric, hydrobromic, sulfuric, nitric, phosphoric; maleic, acetic, salicylic, citric, formic, malonic, succinic acids, and the like. Pharmaceutically compatible salts may also be prepared as alkali metal salts or alkaline earth metal salts, such as sodium salts, potassium salts or calcium salts.

A substance administered according to the invention may comprise a pharmaceutically compatible carrier. The term "carrier" refers to an organic or inorganic component, of a natural or synthetic nature, in which the active component is combined in order to facilitate application. The components of the compositions administered are usually such that no interaction occurs which substantially impairs the desired pharmaceutical efficacy.

The substances administered may contain suitable buffer substances such as acetic acid in a salt, citric acid in a salt, boric acid in a salt and phosphoric acid in a salt.

The substances may, where appropriate, also contain suitable preservatives such as benzalkonium chloride, chlorobutanol, paraben and thimerosal.

The substances may be administered in the form of capsules, tablets, lozenges, solutions, suspensions, syrups, elixirs or in the form of an emulsion or an aerosol, for example.

Substances suitable for parenteral administration usually comprise a sterile aqueous or nonaqueous preparation of the active compound, which is preferably isotonic to the blood of the recipient. Examples of compatible carriers and solvents are Ringer solution and isotonic sodium chloride solution. In addition, usually sterile, fixed oils are used as solution or suspension medium.

### Reference

According to the invention, a "reference" such as a reference sample or reference living organism, or reference living cell may be used to correlate and compare the results obtained in the methods of the invention from a test sample or test living organism or cell. The system used for testing and the system used for assaying a "reference" to obtain a reference value are usually similar but differ in certain variables such as the way they are treated.

A "reference value" can be determined from a reference empirically by measuring a sufficiently large number of references. Preferably the reference value is determined by measuring at least 2, preferably at least 3, preferably at least 5, preferably at least 8, preferably at least 12, preferably at least 20, preferably at least 30, preferably at least 50, or preferably at least 100 references.

### Sample

According to the invention, the cells used for testing may be present within a "sample" which includes test samples and reference samples.

According to the invention, the term "sample" comprises any material including cells, which may be used for testing in the methods according to the invention. Samples or cells used in samples may be obtained from any living organisms, preferably humans and animals. Preferably the living organisms are small laboratory animals such as rats, mice, hamsters, guinea pigs, gerbils, rabbits, zebra fish, drosophila flies or larger mammals, preferably pigs, mini pigs, dogs, cats, apes or humans.

Furthermore, it is possible to use tissues such as visceral fat, abdominal fat or fat from other tissues, muscle, blood, bone, intestine, pancreas, or liver tissue or to use in vitro cultured primary cells or cell lines such as fat, muscle, blood, intestine, pancreas, or liver cells, cell culture medium from primary cells or cell lines, in vitro cultured cells, in vitro cultured tissues, tissue culture medium or cell culture medium as samples or for obtaining samples.

The samples can be obtained by methods known in the art such as venous puncture (blood, serum, plasma), directly collecting urine or collecting urine by use of a catheter. Tissue or cells can be obtained by biopsy or collection during surgery. Blood cells, for example, can be sorted for distinct populations of blood cells such as erythrocytes, macrophages, lymphocytes, B-cells, T-cells, etc. using methods known in the art such as density gradient centrifugation or FACS (fluorescence activated cell sorting), etc. Any kind of the above noted materials, or combinations thereof can be used according to the invention. The sample may be blood, serum, plasma, urine or a tissue sample. Furthermore, the sample or cells may be obtained from a healthy individual, in particular a healthy human or from an individual, in particular a human having a disease associated with a PPAR-gamma receptor, preferably a disease which is treatable by a thiazolidinedione, such as a disease selected from the group consisting of Type II diabetes, insulin resistance, obesity, metabolic syndrome, impaired glucose tolerance, atherosclerosis and coronary heart disease. Furthermore, the sample or cells may be obtained from an individual having a different disease, which is not treatable with a thiazolidinedione and/or associated with a PPAR gamma receptor and which sample or cells therefore may be used as a negative control to distinguish the test sample form said different disease. In a further embodiment, a test sample is obtained from a healthy individual while a reference sample is obtained from an individual afflicted with the above-mentioned diseases or vice versa.

### Methods for determining the level of peptides and/or proteins

If the step of contacting a cell with a candidate substance takes place in vitro in a sample, the level of peptides and/or proteins in the methods of the invention may be determined in the sample or a fraction thereof such as the cell supernatant, or such as certain cell organelles such as nuclei or mitochondria. If the step of contacting a cell with a candidate substance takes place within a living organism, the level of peptides and/or proteins in the methods of the invention may be determined in any sample obtained from said living organism after contacting.

Generally all methods suitable to detect and analyse peptides and proteins can be used in the methods of the invention Preferably mass spectrometric methods, protein chip assays, immunological and molecular biology methods are used.

Preferably peptides or proteins of a molecular weight up to 100 kDa, preferably up to 80 kDa, up to 70 kDa, up to 60 kDa, up to 50 kDa, up to 40 kDa, up to 30 kDa, up to 20 kDa, up to 10 kDa and preferably up to 5 kDa are determined in the methods of the invention.

In certain embodiments of the methods of the invention, high-molecular weight proteins and other biopolymers, which might interfere with the measurement, are removed from the biological sample prior to measurement. Suitable methods for this among others are native or denaturing gel electrophoresis, 2-D gel electrophoresis, liquid chromatography such as anion or cation exchange chromatography, metal chelate affinity chromatography, affinity chromatography, reversed phase chromatography, gas chromatography, capillary chromatography, thin layer chromatography, mass spectrometry, precipitation techniques such as trichloric acetic acid or trifluoric acid or ethanol precipitation, immune precipitation, liquid phase extraction techniques, filtration and other molecular size discriminating techniques such as gel filtration, ultrafiltration using membranes with for example size exclusion limits of 3, 5, 10, 30 or 50 kilo Dalton, density gradient centrifugation and other methods to discriminate molecules according to their density, etc.

Samples may be separated into fractions for determining the level of peptides and proteins and/or different samples and/or different fractions of samples may be analyzed using different measuring arrangements and methods.

Suitable mass spectrometric methods among others are matrix assisted laser desorption ionisation (MALDI), continuous or pulsed electrospray ionization (ESI), SELDI mass spectrometry, FAB mass spectrometry and MS/MS mass spectrometry and related methods such as ionspray or thermospray or massive cluster impact (MCI). The ion sources can be matched with detection formats including linear or non-linear reflection time-of-flight (TOF), single or multiple quadrupole, single or multiple magnetic sector, fourier transform ion cyclotron resonance (FTICR), ion trap, and combinations thereof, e.g. ion-trap/time-of-flight. For ionisation, numerous matrix/wavelength combinations (MALDI) or solvent combinations (ESI) can be used. Other mass spectrometric methods suitable are for example fast atom bombardment (FAB) mass spectrometry, Surface Enhanced Laser Desorption/Ionisation (SELDI) mass spectrometry, isotope coded affinity tag (ICAT) mass spectrometry, or affinity mass spectrometric methods.

Furthermore suitable immunologic methods among others are enzyme linked immuno assays (ELISA), sandwich, direct, indirect, or competitive ELISA assays, enzyme-linked immunospot assays (ELISPOT), radio immuno assays (RIA), flow cytometry assays (FACS = fluorescence activated cell sorting), immunohistochemistry, Western blot, fluorescence resonance energy transfer (FRET) assays, protein-chip assays using for example antibodies, antibody fragments, receptors, ligands, or other binding agents specific for peptides or proteins.

Further methods such as nuclear magnetic resonance (NMR), fluorometry, colorimetry, radiometry, luminometry, or other spectrometric methods, liquid chromatography, capillary chromatography, thin-layer chromatography, surface plasmone resonance (SPR) measurements (BIAcore), one- or two-dimensional gel electrophoresis, etc. can be used to detect peptides and proteins.

### Peptide/protein pattern

The determination of the level of peptides and/or proteins in the methods of the invention may include a generation of protein/peptide patterns which patterns comprise at least two distinct signals and preferably a comparison of protein/peptide patterns generated. A comparison of protein/peptide patterns may reflect different amounts of peptides and/or proteins to be tested in the methods of the invention.

Such protein/peptide pattern preferably comprises two or more, preferably 4 or more, more preferably 6 or more, 8 or more, 10 or more, 15 or more, even more preferably 20 or more, 30 or more, 40 or more, 50 or more, 60 or more, 70 or more, 80 or more, 90 or more, 100 or more, 120 or more, 140 or more, 160 or more, 180 or more, 200 or more, 250 or more, 300 or more, 400 or more, 500 or more signals for peptides and/or proteins. The upper limit of said signals is not particularly restricted as long as the number of said signals does not exceed the resolution limit of the assay system and the available computing power to calculate the correlations. An upper limit may be for example not more than 20, 40, 60, 80, 100, 150, 200, 250, 300, 400, 450, 500, 1000, 2000, 5000 or 10000 signals depending on the assay system used in the methods of the invention. In certain embodiments of the methods of the invention, only a part or fraction of the peptides and/or proteins in a sample is used for creating a protein/peptide pattern. In these embodiments the peptides and/or proteins may be subjected to separation means such as chromatographic separations before creating a protein/peptide pattern. For evaluation of such patterns preferably at least one of the following mathematical methods to calculate correlations is used: "Pearson Product-Moment Correlation", "Spearman's Rank-Order Correlation", "Kendall's Tau", "Kendall's Coefficient of Concordance", "Goodman and Kruskal's Gamma", "Manhattan distance", "Euclidean distance" and "Minimal Spanning Tree Diameter". Most preferably "Spearman's Rank-Order Correlation" is used.

The present invention is described in detail by the following examples, which are used only for illustration purposes and are not meant to be limiting in any way. Owing to the description and the examples, further embodiments which are likewise included in the invention are accessible to the skilled person.

### Examples

### Example 1: Fat cell differentiation, stimulation and sample preparation:

Cells of the fibroblast cell line 3T3-L1 (American Tissue Type Collection, Manassas, VA, USA) were cultured in Dulbecco's Modified Eagle's Medium (Dulbecco's Modified Eagle's Medium supplemented with 1 mM Na-pyruvate, 4 mM L-glutamine, ; subsequently termed DMEM) and with 10 % v/v fetal calf serum (FCS). Two day post-confluent 3T3-L1 cells were differentiated into adipocytes (fat cells) by culturing in DMEM with 10 % FCS and supplemented with 1 µg/mL isobutyl-1-methylxanthine (IBMX), 0.25 µmol/L dexamethasone and 1 µg/mL insulin for 3 days. After additional two days in DMEM with 10 % FCS supplemented with 1 µg/mL insulin only, the cells were maintained in DMEM with 10 % FCS until more than 90 % of the cells had accumulated lipid droplets, which was monitored by light microscopy. On day 10, a first stimulus of 1 µM Troglitazone was added to all cells, except to those cells (negative control) which subsequently did not get a second stimulus. The first stimulus was incubated with the cells for 40 h. The cells were rinsed with phosphate buffered saline and for the second stimulus the culture medium was changed to DMEM without FCS (negative control), DMEM without FCS but with 1 µM Troglitazone (Calbiochem, Darmstadt, Germany), or with 10 nM insulin (recombinant, human insulin, Sigma, München, Germany), or with both, 1 µM Troglitazone and 10 nM Insulin. After incubation for 8 h, the conditioned medium was harvested, centrifuged for 10 min at 300 x g and sterile filtered using a Minisart 0,2 µm filter unit (Sartorius, Göttingen, Germany). From each sample one aliquot for the determination of triglycerides and total protein was stored at - 20 °C, and another aliquot for the determination of lactate dehydrogenase (LDH) activity was stored at 6 °C. The remainder of each sample was acidified to a pH value of about 2 to 3 using concentrated HCl (5µl/mL), stored at - 80 °C and used for generating peptide maps and for sequencing of proteins and peptides (cf. Examples 3 to 5).

### Example 2: Measurement of total protein, LDH activity, adiponectin, triglycerides and MCP-1

The following assays were performed according to standard methods as known in the art or according to the manufacturer's instructions for the assay kit used to rule out that differences measured between the treatment groups were due to different sample quantities (measurement of total protein), different cell viability (measurement of LDH activity), or different grade of differentiation of the cells (measurement of adiponectin and triglycerides).

The determination of total protein was done using "Roti®-Quant", a Bradford-type protein assay (Roth, Karlsruhe, Germany). Serum free DMEM was used as negative control. All samples were adjusted for equal protein concentrations to enable a direct comparison of the mass spectrometric results obtained with these samples.

LDH, a cytosolic enzyme released upon cell lysis, was determined using the "Cytotoxicity Detection Kit (LDH)" (Roche Molecular Biochemicals, Mannheim, Germany). The serum free cell culture supernatants of stimulated (with Troglitazone, insulin or both) and control cells (no stimulus) were analyzed. Serum free medium was used as negative control. The results of the LDH assay indicated that the fat cells of all 4 treatment groups were equally viable and that none of the stimuli used had toxic effects towards the fat cells.

To confirm that the differentiation into fat cells was successful triglycerides and adiponectin were measured, and the cells were visually inspected for intracellular accumulation of fat droplets using light microscopy. Usually 70 to 80 % of the cells were differentiated into fat cells. Adiponectin was measured using a commercially available Adiponection ELISA (B-Bridge International Inc., Sunnyvale, CA, USA). DMEM medium or conditioned DMEM medium from control cells (not differentiated 3T3-L1 cells) was used as negative control. Triglycerides were measured using the "Fluitest® TG" triglyceride assay (Biocon Diagnostik, Vöhl/Marienhagen, Germany). This assay is an enzymatic, colorimetric assay based on the "Trinder reaction". DMEM medium was used as negative control. As shown in Fig. 2A, the samples of all 4 treatment groups contained about the same concentration of adiponectin indicating that the cells of all 4 treatment groups had an equal level of differentiation. This was also confirmed by the results obtained from the triglyceride assay (data not shown).

Monocyte Chemoattractant Protein 1 (MCP-1) belongs to the CC chemokine family of inflammatory and immunoregulatory cytokines and is upregulated by insulin, which is one of the know pro-inflammatory activities of insulin. Therefore, measuring the effects of insulin and/or Troglitazone towards the secretion of MCP-1 allows to determine the overall anti-inflammatory activity of Troglitazone. Determination of MCP-1 in cell culture supernatants of stimulated and non-stimulated, differentiated 3T3-L1 fat cells was done using a sandwich ELISA (R&D Systems GmbH, Wiesbaden, Germany). DMEM medium was used as negative control. As shown in Fig. 2B, insulin alone increases the concentration of MCP-1. However, Troglitazone alone or in combination with insulin decreases the concentration of MCP-1 to below the level of non-stimulated differentiated fat cells indicating an anti-inflammatory effect of Troglitazone. These results confirm that insulin and Troglitazone showed the expected inflammatory and. anti-inflammatory activities, respectively, on the fat cells tested indicating that a cell stimulation in general can be achieved in this way.

### Example 3: Liquid chromatography of the samples

The separation of peptides and proteins was done using a Source 5RPC, 4,6 x 150 mm reverse phase chromatography column (Amersham Biosciences Europe GmbH, Freiburg, Germany). Buffer A was 0.06 % (v/v) trifluoroacetic acid (TFA) in distilled water and buffer B was 0.05 % (v/v) TFA, and 80 % (v/v) acetonitrile in distilled water. Chromatography was performed at 33°C using a HP 1100 HPLC with a micro flow cell (both supplied by Agilent Technologies, Böblingen, Germany). Prior to chromatography, the samples (total volume of 5.5 mL) were centrifuged at 4°C and 15000 x g for 10 minutes and finally 5 mL of the supernatant thus obtained from the sample was loaded onto the chromatography column. If less starting material was desired, the supernatant obtained from the sample was diluted using a 0.1 % (v/v) stock solution of trifluoroacetic acid. Buffers A and B were mixed in various ratios during chromatography.. The chromatography conditions are as follows wherein only the amount in percentage of buffer B is stated and the amount in percentage of buffer A is 100 % minus % buffer B (v/v):
- 5 % buffer B for 1 min;
- a linear gradient from 5 to 50 % buffer B in 44 min;
- a linear gradient from 50 to 100 % buffer B in 4 min;
- 100 % buffer B for 4 min.
The flow rate was 500 µL/min and 96 fractions each containing 0.25 mL are collected during the gradients from 5 to 100 % buffer B.

### Example 4: Mass spectrometry of the samples

For mass spectrometric analysis, typical positive ion spectra of peptides were measured using a MALDI-TOF (matrix-assisted laser desorption ionization time of flight) mass spectrometer. For MALDI sample preparation, α-cyano-4-hydroxycinnamic acid was used as matrix and 6-desoxy-L-galactose was used as co-matrix, dissolved in acetonitrile containing 0.1% TFA. The sample which has been fractionated by reverse phase chromatography, and lyophilized was dissolved in 15 µL of matrix solution (10 g/L α-cyano-4-hydroxycinnamic acid and 10 g/L L(-)fucose dissolved in a solvent mixture consisting of acetonitrile, water, trifluoroacetic acid (TFA) and acetone in a ratio of 49:49:1:1 by volume). 0.3 µL of this sample solution (= equivalent to 100 µL of the original sample) were transferred to a MALDI carrier plate, and the dried sample is analyzed in a Voyager-DE STR MALDI mass spectrometer (PerSeptive Biosystems). The measurement was performed in linear mode with delayed extraction^{™}. A MALDI-TOF mass spectrometer can be employed to quantify peptides and/or proteins such as, for example, the peptides and proteins described herein if these peptides and/or proteins are present in a concentration which is within the dynamic measurement range of the mass spectrometer, thus avoiding detector saturation. Fig. 3A to 3C show the results of this example. The concentration of a peptide representing a fragment of Fn14 (amino acid residues 28 to 88) was increased in the supernatant of stimulated versus non-stimulated cells, more increased in the supernatant of insulin-stimulated compared to Troglitazone-stimulated cells, and most increased in the supernatant of cells stimulated with insulin and Troglitazone. A similar fragment of Fn14, representing amino acid residues 28 to 87 showed in all four groups results very similar to those obtained with respect to the first Fn14 fragment. Fig. 3B shows that the concentration of a fragment of the High Mobility Group A 1 (HMGA) protein, representing amino acid residues 73 to 93, was increased in the supernatant of all stimulated treatment groups compared to the non-stimulated control. However, stimulation with Troglitazone resulted in the highest concentration of this fragment, the stimulation with insulin resulted in a lower increase and the combination of insulin and Troglitazone resulted in the lowest increase relative to the non-stimulated cells. Fig. 3C shows that the concentration of Apelin-13, a fragment of Apelin, was slightly increased in the supernatant of all stimulated cells compared to non-stimulated cells. In the supernatant of cells stimulated with Troglitazone and insulin, the concentration of Apelin-13 is slightly more increased compared to cells stimulated with either Troglitazone or insulin alone.

### Example 5: Peptide identification

Detection of concentration differences of individual peptides and/or proteins was achieved by calculation of subtractive peptide display maps and correlation analysis. Selected proteins and peptides, which appear in peptide display maps are analyzed by ESI-qTOF sequencing (PE Applied Biosystems, Framingham, USA). Peptide ions were selected in the mass spectrometer on the basis of their specific m/z (mass/charge) values in a manner known to the skilled worker. These selected ions were then fragmented by supplying collision energy (20 - 40 eV) with an collision gas, e.g. helium or nitrogen, the resulting mass spectrometric fragments of the peptides and/or proteins were detected in the mass spectrometer in an integrated analysis unit, and corresponding m/z values were determined (principle of tandem mass spectrometry). The fragmentation behavior of peptides and/or proteins enables the unambiguous identification of the peptides and/or proteins. The mass-spectrometric analysis can be done, for example, by Quadrupol-TOF (time of flight) sequencing (QStar-Pulsar model) or ESI-qTOF sequencing (both from PE Applied Biosystems, Framingham, USA). The resulting peptide/protein fragment spectra are detected using nanoSpray in the product ion scan mode (spray voltage 950 V, collision energy 20-40 eV). Up to 200 scans per sample are accumulated. Charge state deconvolution is performed using the Bayesian reconstruct tool of the BioAnalyst program package (PE Applied Biosystems), and deisotoping is achieved by means of the Voyager 5.1 software (PE Applied Biosystems). The mass spectra are saved in a MASCOT generic file format, and submitted to the MASCOT19 database search engine (Matrix Science, London, UK). Searched databases, for example, can be SwissProt (Version 39.6, www.expasy.ch) and MSDB (Version 010721, EBI, Europe). In addition, this peptide sequencing method allows the identification of amino acid modifications such as phosphorylation, acetylation or hydroxylation. By using the methods and means described above, the amino acid sequence and origin of the peptides described in example 4, were identified.

## Claims

1. A method for identifying a therapeutic comprising the step of contacting a cell with a candidate substance and determining the level of at least one peptide and/or protein selected from the group consisting of Fn14, HMGA1, Apelin, fragments thereof, and variants thereof.

2. The method of claim 1 wherein said cell is responsive to treatment with a thiazolidinedione.

3. The method of claim 1 or 2, wherein said cell expresses a PPAR-gamma receptor.

4. The method of any one of claims 1 to 3, wherein said cell is an adipocyte.

5. The method of any one of claims 1 to 4 wherein said candidate substance is a compound or a composition of compounds.

6. The method of any one of claims 1 to 5 wherein said therapeutic is a substance having at least one functional property of a thiazolidinedione or having analogous activity to a thiazolidinedione.

7. The method of any one of claims 1 to 6 wherein an increase in the level of said at least one peptide and/or protein and/or a decrease in the level of said at least one peptide and/or protein if a cell has been contacted with said candidate substance relative to if a cell has not been contacted with said candidate substance indicates that the candidate substance is a therapeutic useful in the treatment of a disease, in particular a disease associated with a PPAR-gamma receptor.

8. The method of any one of claims 1 to 7 wherein an increase in the level of said at least one peptide and/or protein if a cell has been contacted with said candidate substance relative to if a cell has not been contacted with said candidate substance indicates that the candidate substance has at least one functional property of a thiazolidinedione and/or is useful in the treatment of a disease treatable with a thiazolidinedione and/or associated with a PPAR-gamma receptor.

9. The method of any one of claims 1 to 8 wherein said contacting takes place in vitro in a sample or in vivo in a living cell or in vivo within a living organism.

10. The method of any one of claims 1 to 9 wherein the level of said at least one peptide and/or protein is compared with the level of said at least one peptide and/or protein in a reference or with a predetermined reference value for said at least one peptide and/or protein.

11. The method of claim 10 wherein in said reference a cell (i) has not been contacted with said candidate substance, and/or (ii) has been contacted with a thiazolidinedione or a combination of a thiazolidinedione and said candidate substance, and/or (iii) has been contacted with said candidate substance, a thiazolidinedione or a combination of a thiazolidinedione and said candidate substance for a different period of time and/or in a different concentration.

12. The method of any one of claims 1 to 10 wherein said contacting takes place within a living organism by administering said candidate substance to said living organism and the level of said at least one peptide and/or protein is compared with the level of said at least one peptide and/or protein in at least one further living organism (i) to which said candidate substance has not been administered, and/or (ii) to which a thiazolidinedione or a combination of a thiazolidinedione and said candidate substance has been administered, and/or (iii) in which said contacting with said candidate substance, a thiazolidinedione or a combination of a thiazolidinedione and said candidate substance took place for a different period of time, and/or (iv) to which a different dose of said candidate substance has been administered, and/or (v) to which said candidate substance has been administered by different means of administration.

13. A method for determining whether a living organism is responsive to treatment with a substance having at least one functional property of a thiazolidinedione comprising the step of administering said substance to said living organism and determining the level of at least one peptide and/or protein selected from the group consisting ofFnl4, HMGA1, Apelin, fragments thereof, and variants thereof in a sample obtained from said living organism.

14. The method of claim 13 wherein said sample is selected from the group consisting of blood, serum, plasma, and urine.

15. The method of claim 13 or 14 wherein an increase in the level of said at least one peptide and/or protein and/or a decrease in the level of said at least one peptide and/or protein in a living organism to which said substance has been administered relative to a living organism to which said substance has not been administered indicates that said substance is effective in treating a disease, in particular a disease associated with a PPAR-gamma receptor.

16. The method of any one of claims 13 to 15 wherein an increase in the level of said at least one peptide and/or protein in a living organism to which said substance has been administered relative to a living organism to which said substance has not been administered indicates that said substance is effective in the treatment of a disease treatable with a thiazolidinedione and/or associated with a PPAR-gamma receptor.

17. The method of any one of claims 13 to 16 wherein said level of said at least one peptide and/or protein is compared with the level of said at least one peptide and/or protein in at least one further living organism (i) to which said substance has not been administered, and/or (ii) to which a thiazolidinedione or a combination of a thiazolidinedione and said substance has been administered, and/or (iii) which has been subjected to said substance, a thiazolidinedione or a combination of a thiazolidinedione and said substance for a different period of time, and/or (iv) to which a different dose of said substance has been administered, and/or (v) to which said substance has been administered by different means of administration.

18. The method of any one of claims 1 to 17 wherein said determining comprises determining the level of Fn14 and/or at least one fragment thereof and/or at least one variant thereof and/or HMGA1 and/or at least one fragment thereof and/or at least one variant thereof and/or Apelin and/or at least one fragment thereof and/or at least one variant thereof.

19. The method of any one of claims 1 to 18, wherein said fragment of Fn14 corresponds to amino acid residues 28 to 84, 28 to 83, 28 to 87 or 28 to 88 of a Fn14 protein and preferably has an amino acid sequence selected from the group consisting of SEQ ID NO:7 to 10 of the sequence listing.

20. The method of any one of claims 1 to 18, wherein said fragment of HMGA1 corresponds to amino acid residues 73 to 95 of a HMGA 1 protein and preferably has the amino acid sequence according to SEQ ID NO:11 of the sequence listing.

21. The method of any one of claims 1 to 18, wherein said fragment of Apelin corresponds to amino acid residues 23 to 48, 27 to 41, or 65 to 77 of an Apelin protein and preferably has an amino acid sequence selected from the group consisting of SEQ ID NO:12 to 14 of the sequence listing.

22. The method of any one of claims 1 to 21, wherein said determining of the level of said at least one peptide and/or protein comprises a determination of the relative or absolute quantity of said at least one peptide and/or protein.

23. The method of any one of claims 1 to 22, wherein said determining of the level of said at least one peptide and/or protein is performed using mass spectrometric methods, or mass spectrometric methods in combination with separation methods.

24. The method of claim 23, wherein said separation methods are selected from the group consisting of gel electrophoresis, liquid chromatography, gas chromatography, capillary chromatography, thin layer chromatography, mass spectrometry, precipitation techniques, liquid phase extraction techniques, filtration and other molecular size discriminating techniques, density gradient centrifugation and other methods to discriminate molecules according to their density.

25. The method of claim 23 or 24, wherein said mass spectrometric methods are selected from the group consisting of MALDI mass spectrometry, ESI mass spectrometry, SELDI mass spectrometry, FAB mass spectrometry and MS/MS mass spectrometry.

26. The method of any one of claims 2 to 25 wherein said thiazolidinedione is selected from the group consisting of Rosiglitazone, Pioglitazone, Troglitazone, MCC-555, MK-0767, TZD18, Balaglitazone, Farglitazar, Ciglitazone, and Darglitazone.

27. The method of any one of claims 7 to 12 and 15 to 26 wherein said disease is selected from the group comprising Type II diabetes, insulin resistance, impaired glucose tolerance, obesity, metabolic syndrome, cardiovascular diseases and atherosclerosis.

28. The method of any one of claims 1 to 27, wherein Fn14 comprises an amino acid sequence according to SEQ ID NO:1 or 2 of the sequence listing.

29. The method of any one of claims 1 to 27, wherein HMGA1 comprises an amino acid sequence according to SEQ ID NO:3 or 4 of the sequence listing.

30. The method of any one of claims 1 to 27, wherein Apelin comprises an amino acid sequence according to SEQ ID NO:5 or 6 of the sequence listing.

31. A peptide having an amino acid sequence selected from the group consisting of SEQ ID NO:7 to 13 of the sequence listing, or a variant thereof.

32. A nucleic acid encoding a peptide having an amino acid sequence selected from the group consisting of SEQ ID NO:7 to 13 of the sequence listing, or a variant thereof.

33. A recombinant nucleic acid comprising a nucleic acid or variant thereof of claim 32.

34. An antibody which binds specifically to a peptide having an amino acid sequence selected from the group consisting of SEQ ID N0:7 to 14 of the sequence listing, or a variant thereof.
